# EUROPÄISCHE PATENTSCHRIFT

(11) **EP 0 444 585 B1**
(45) Veröffentlichungstag und Bekanntmachung des Hinweises auf die Patenterteilung: **13.07.1994**
(21) Anmeldenummer: 91102787.8
(22) Anmeldetag: 26.02.1991
(51) Int. Cl.: C02F 3/12, C02F 3/30

(54) **Klärbehälter**
Clarification vessel
Cuve de clarification

(30) Priorität: 26.02.1990 DE 4005975
(43) Veröffentlichungstag der Anmeldung: 04.09.1991
(73) Patentinhaber: Ballies, Uwe Werner, Dr., 24103 Kiel (DE)
(72) Erfinder: Ballies, Uwe Werner, Dr., 24103 Kiel (DE)
(74) Vertreter: Tönnies, Jan G., Dipl.-Ing.

(56) Entgegenhaltungen:
- EP-A- 49 954
- DE-A- 839 245
- US-A- 278 547
- US-A- 396 507
- US-A- 4 581 143
- Das Gas-UND WASSERFACH, Bd. 130, nr. 7, Juli 1989, München, seiten 334-340 H:RENNER ET AL. "EINE BIOLOGISCHE HAUSKLÄRANLAGE OHNE FREMDERNERGIE"

## Beschreibung

Die Erfindung betrifft einen Klärbehälter zum biologischen Reinigen von Wasser mit den Merkmalen des Oberbegriffs des Anspruchs 1.

Es ist bekannt, daß Mikroorganismen, insbesondere aerobe und anerobe Bakterienkulturen zur biologischen Klärung von Wasser genutzt werden können. Dies gilt insbesondere für die Klärung von in der Landwirtschaft anfallender Jauche sowie der Reinigung des durch Stoffwechselprodukte verschmutzten Wassers von Aquakulturen, also Anlagen zur Zucht von Fischen oder Schalentieren.

Der aus der DE-GM 69 060 029 vorbekannte Klärbehälter zum biologischen Reinigen von Wasser ist relativ kompliziert und energieaufwendig.

Der Erfindung liegt damit die Aufgabe zugrunde, einen Klärbehälter zum biologischen Reinigen von Wasser zu schaffen, der einfach aufgebaut ist und dessen Energiebedarf gering ist.

Erfindungsgemäß wird diese Aufgabe durch die im kennzeichnenden Teil des Anspruchs 1 angegebenen Merkmale gelöst.

Um einen einstellbaren Gegendruck zu erzeugen, kann der Wasserauslaß mit einem höhenverstellbaren Steigrohr versehen sein. Alternativ kann jedoch auch der Querschnitt des Wasserauslasses einstellbar ausgebildet sein.

Eine bevorzugte Ausführungsform zeichnet sich dadurch aus, daß das Ventil in dem Luftauslaß ein Druckventil mit einer erheblichen Hysterese ist.

Es kommt jedoch auch eine Ausbildung mit einem getaktet betriebenen Stellventil (Drehventil) in Betracht.

Es wird vorgeschlagen, daß die Bakterienkulturen von einer für die abzubauenden Stoffwechselprodukte durchlässigen, für die Bakterien jedoch undurchlässigen Membrane gehalten werden. Dem Fachmann sind derartige Membranen bekannt.

Eine bevorzugte Ausführungsform zeichnet sich dadurch aus, daß ein zweites Rückschlagventil in dem Wasserauslaß vorgesehen ist.

Bei Vorsehung eines derartigen zweiten Rückschlagventils wird vermieden, daß bereits gereinigtes Wasser durch den Wasserauslaß der in den Behälter eingesogen wird.

Es kann vorgesehen sein, daß der Behälter innerhalb des das zu reinigende Wasser aufnehmenden Tanks angeordnet ist.

Zur Bewahrung der durch die exothermen Umsetzungen in dem Behälter entstehenden Wärme wird weiter vorgeschlagen, daß der Behälter thermisch isoliert ist.

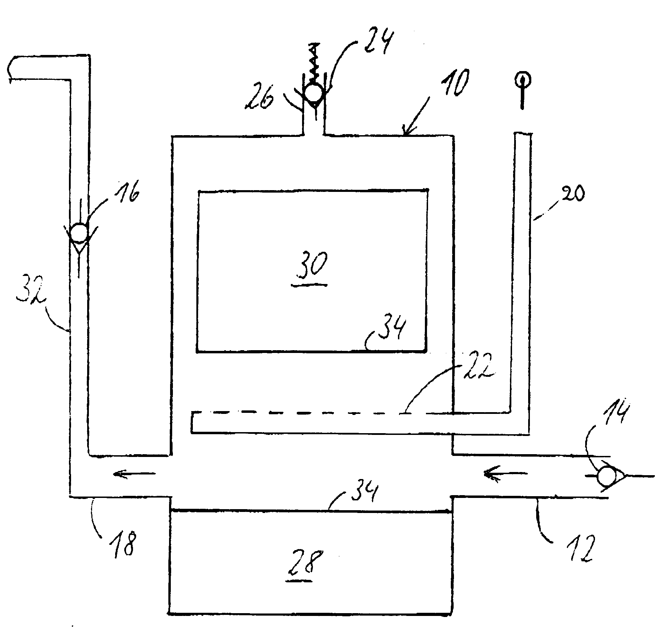

Die Erfindung wird im folgenden anhand einer Zeichnung erläutert. Dabei zeigt die einzige Figur eine schematische Schnittdarstellung des Behälters.

Der luftdicht abgeschlossene Behälter 10 - beispielsweise ein Kunststoff-Faß - ist mit einem Wassereinlaß 12 versehen. Dieser Wassereinlaß 12 weist ein Rückschlagventil 14 auf, das ein Rückströmen von Wasser aus dem Behälter 10 verhindert. Der Behälter 10 ist weiter mit einem dem Wassereinlaß 12 gegenüberliegend angeordneten Wasserauslaß 18 versehen, in dem bei dem dargestellten Ausführungsbeispiel ein weiteres Rückschlagventil 16 angeordnet ist. In dem Deckel des Behälters 10 ist ein Luftauslaß 26 vorgesehen, in dem ein Ventil 24 angeordnet ist. Eine sich durch den Behälter 10 erstreckende Luftzuführung 22 wird über einen Lufteinlaß 20 mit einer - nicht gezeigten - Luftpumpe verbunden.

Unterhalb des Wassereinlasses 12 wird eine anaerobe Bakterienkultur 28 gehalten, oberhalb der Luftzuführung 22 eine aerobe Bakterienkultur 30. Dabei ist die räumliche Anordnung dieser Bakterienkulturen 28, 30 nicht entscheidend, von Bedeutung ist lediglich, daß die anerobe Bakterienkultur außerhalb des Bereichs des aus der Luftzuführung 22 austretenden Luftstroms und die aerobe Bakterienkultur innerhalb des Bereichs des aus der Luftzuführung austretenden Luftstroms angeordnet ist.

An den Wasserauslaß 14 ist ein höhenverstellbares Steigrohr 32 angeschlossen.

Das Ventil 24 in dem Luftauslaß 26 kann als ein zeitgetakt betriebenes Stellventil ausgebildet sein, es kann sich dabei jedoch auch um ein Druckventil handeln, das eine erhebliche Hysterese hat, also um ein Druckventil, das bei Überschreiten eines bestimmten ersten Drucks öffnet und erst bei Erreichen des athmosphärischen Drucks wieder schließt. Alternativ kommt auch eine Steuerung des Ventils über den Wasserspiegel in Betracht. Für die Steuerung ist von Bedeutung, daß die aerobe Bakterienkultur 30 regelmäßig von dem Wasser benetzt wird und sodann wieder trockenfällt.

Dem Fachmann sind verschiedene Möglichkeiten des Haltens der Bakterienkulturen 28, 30 bekannt, bei dem gezeigten Ausführungsbeispiel werden die Bakterienkulturen 28, 30 von Membranen 34 gehalten, die für den in dem Wasser schwebenden abzubauenden Stoffwechselprodukte durchlässig, für die Bakterien jedoch undurchlässig sind.

Die - zeichnerisch nur angedeutete - Luftpumpe wird kontinuierlich betrieben, so daß durch den Lufteinlaß 20 ständig Luft in den Behälter 10 eingebracht wird. Die Luft tritt durch die Luftzuführung 22 aus und durchströmt die anaerobe Bakterienkultur 28, die so mit Sauerstoff versorgt wird. Bei geschlossenem Ventil 24 bildet sich im oberen Bereich des Behälters 10 ein Luftkissen aus, das ständig zunimmt. Da das Wasser in dem Behälter 10 nicht durch den Wassereinlaß 12 rückströmen kann, wird es durch den Wasserauslaß 18 und das Steigrohr 32 aus dem Behälter 10 herausgedrückt und ab- oder in einen Tank rückgeführt.

In regelmäßigen Zeitabständen oder aber - bei Vorsehung eines Druckventils - wird das Ventil 24 geöffnet, das Luftkissen wird abgeblasen. Zu diesem Zeitpunkt strömt Wasser durch den Wassereinlaß 12 nach, nach Erreichen eines Wasserstandes, der demjenigen in dem Tank, aus dem das zu reinigende Wasser nachgeführt wird, erreicht wird, besteht ein Druckausgleich, das Ventil 24 wird wieder geschlossen. Es baut sich wieder in dem Behälter 10 ein Druck auf, der zu einem Ausdrücken des Wassers aus dem Wasserauslaß 18 durch das Steigrohr 32 führt.

Bei der hier vorgeschlagenen Ausbildung eines Klärbehälters ist lediglich eine Antriebsquelle, nämlich die Luftpumpe vorgesehen. Die durch die Luftzuführung 22 austretende Luft versorgt zum einen die zunächst unter Wasser stehende aerobe Bakterienkultur mit Sauerstoff. Nach Ausbildung des Luftkissens in dem oberen Bereich des Behälters wird die aerobe Bakterienkultur 30 wenigstens teilweise der Luft ausgesetzt, wobei der Luftdruck jedoch erhöht ist. Dies bewirkt eine besonders gute Versorgung der aeroben Bakterienkultur mit Sauerstoff.

Die anaerobe Bakterienkultur dagegen liegt außerhalb des Bereiches des aus der Luftzuführung 22 austretenden Luftstroms, sie wird also nicht mit Sauerstoff versorgt. Die Stoffwechselprodukte in dem Wasser werden zunächst von der unten angeordneten anaeroben Bakterienkultur umgesetzt, die Umsetzungsprodukte werden sodann nach oben ausgetrieben und werden in dem Bereich der aeroben Bakterienkultur 30 weiter umgesetzt. Auf eine eingehende Beschreibung der mikrobiologischen und chemischen Prozesse wird hier verzichtet, da diese nicht Gegenstand der Erfindung sind.

Es versteht sich, daß Sorge dafür getragen werden muß, daß bei Verwendung eines Druckventils mit erheblicher Hysterese dieses erst dann anspricht, wenn bereits geraume Zeit Wasser durch den Wasserauslaß 18 herausgefördert worden ist. Dies kann beispielsweise durch einen dem Steigrohr 32 nachgeschalteten Behälter bewirkt werden, dessen Austrittsöffnung geringer ist als die Eintrittsöffnung, so bei gefülltem weiteren Behälter ein erhöhter Druck, bei dessen Erreichen das Druckventil 24 anspricht, gefüllt wird.

Es können mehrere derartiger Behälter kaskadenartig hintereinander angeordnet werden. Einige oder alle diese Klärbehälter können auch in dem Tank, der das zu reinigende Wasser aufnimmt, angeordnet sein.

Bei Verwendung zur Reinigung von Aquakulturen wird ein geschlossener Kreislauf gebildet.

Bei der Verwendung zur Klärung von Jauche und dgl. ist diese wegen der hohen Konzentration der Gülle vor Einbringen in den Behälter 10 zu verdünnen, das aus dem Wasserauslaß 18 austretende Wasser kann sodann erneut zur Verdünnung nachgeführter Gülle verwendet werden.

Die hier vorgeschlagene Ausbildung ist konstruktiv sehr einfach, es ist lediglich ein Antriebsmittel, nämlich die Luftpumpe, erforderlich. Der hier vorgeschlagene Behälter ist weiter energetisch sehr günstig.

## Patentansprüche

1. Klärvorrichtung mit einem Behälter (10), der mit einem Wassereinlaß (12), einem Wasserauslaß (18), einem Lufteinlaß (20) und einer Luftzuführung (22) versehen ist, wobei in dem Behälter (10) außerhalb des Bereichs des aus der Luftzuführung (22) austretenden Luftstroms eine anaerobe Bakterienkultur (28) gehalten wird, und innerhalb des Bereichs des aus der Luftzuführung (22) austretenden Luftstroms eine aeroben Bakterienkultur (30) gehalten wird,
dadurch gekennzeichnet, daß
- der Behälter (10) luftdicht verschlossen ist,
- der Wassereinlaß (12) mit einem ersten Rückschlagventil (14) versehen ist,
- ein mit einem Ventil (24) versehener Luftauslaß (26) vorgesehen ist, und
- Mittel zum zeit-, wasserspiegel- oder druckgesteuerten Öffnen des Luftauslasses (26) vorhanden sind.

2. Klärbehälter nach Anspruch 1, daß der Wasserauslaß (18) mit einem höhenverstellbaren Steigrohr (32) versehen ist.

3. Klärbehälter nach Anspruch 1 oder Anspruch 2, dadurch gekennzeichnet, daß das Ventil (24) in dem Luftauslaß (26) ein Druckventil mit einer erheblichen Hysterese ist.

4. Klärbehälter nach einem der vorangehenden Ansprüche, dadurch gekennzeichnet, daß die Bakterienkulturen (28, 30) von einer für die abzubauenden Stoffwechselprodukte durchlässigen, für die Bakterien jedoch undurchlässigen Membrane (34) gehalten werden.

5. Klärbehälter nach einem der vorangehenden Ansprüche, dadurch gekennzeichnet, daß ein zweites Rückschlagventil (16) in dem Wasserauslaß (18) vorgesehen ist.

6. Klärbehälter nach einem der vorangehenden Ansprüche, gekennzeichnet durch seine Anordnung innerhalb des das zu reinigende Wasser aufnehmenden Tanks.

7. Klärbehälter nach einem der vorangehenden Ansprüche, dadurch gekennzeichnet, daß der Behälter (10) thermisch isoliert ist.

## Claims

1. Clarifier with a container (10), which is provided with a water inlet (12), a water outlet (18), an air inlet (20) and an air supply (22), an anaerobic bacterial culture (28) being kept in the container (10) outside the area of the air flow passing out of the air supply (22) and an aerobic bacterial culture (30) is kept within the area of the air flow passing out of the air supply (22), characterized in that the container (10) is closed in airtight manner, the water inlet (12) is provided with a first check valve (14), an air outlet (26) provided with a valve (24) is present and there are means for the time-, water level- or pressure-controlled opening of the air outlet (26).

2. Clarifier according to claim 1, characterized in that the water outlet (18) is provided with a vertically adjustable riser (32).

3. Clarifier according to claim 1 or 2, characterized in that the valve (24) in the air outlet (26) is a pressure valve with a considerable hysteresis.

4. Clarifier according to any one of the preceding claims, characterized in that the bacterial cultures (28, 30) are retained by a diaphragm (34) which is permeable for the metabolites to be decomposed, but impermeable for the bacteria.

5. Clarifier according to any one of the preceding claims, characterized in that there is a second check valve (16) in the water outlet (18).

6. Clarifier according to any one of the preceding claims, characterized by its arrangement within the tank receiving the water to be purified.

7. Clarifier according to any one of the preceding claims, characterized in that the container (10) is thermally insulated.

## Revendications

1. Dispositif d'épuration, avec un récipient (10), pourvu d'une admission d'eau (12), d'une évacuation d'eau (18), d'une admission d'air (20) et d'une amenée d'air (22), une culture bactérienne (28) anaérobique étant entretenue dans le récipient (10), à l'extérieur de la zone du courant d'air sortant de l'amenée d'air (22), et une culture bactérienne (30) aérobique étant entretenue à l'intérieur de la zone du courant d'air sortant de l'amenée d'air (22),
caractérisé en ce que
- le récipient (10) est obturé de façon étanche à l'air,
- l'admission d'eau (12) est pourvue d'un premier clapet anti-retour (14),
- une évacuation d'air (26), pourvue d'une soupape (24), est prévue, et
- des moyens d'ouverture de l'évacuation d'air (26) sont installés, opérant une commande en fonction du temps, du niveau de l'eau ou de la pression.

2. Récipient d'épuration selon la revendication 1, caractérisé en ce que l'évacuation d'eau (18) est pourvue d'une colonne montante (32) réglable en hauteur.

3. Récipient d'épuration selon la revendication 1 ou la revendication 2, caractérisé en ce que la soupape (24) montée dans l'évacuation d'air (26) est une soupape de pression présentant un hystérésis notable.

4. Récipient d'épuration selon l'une des revendications précédentes, caractérisé en ce que les cultures bactériennes (28, 30) sont retenues par une membrane (34) perméable aux produits d'échange de matière devant être détruits, tout en étant cependant imperméable aux bactéries.

5. Récipient d'épuration selon l'une des revendications précédentes, caractérisé en ce qu'un deuxième clapet anti-retour (16) est prévu dans l'évacuation d'eau (18).

6. Récipient d'épuration selon l'une des revendications précédentes, caractérisé par le fait de le disposer à l'intérieur du réservoir recevant l'eau à épurer.

7. Récipient d'épuration selon l'une des revendications précédentes, caractérisé en ce que le récipient (10) est isolé thermiquement.
